# EUROPÄISCHE PATENTSCHRIFT

(11) **EP 1 495 749 B1**
(45) Veröffentlichungstag und Bekanntmachung des Hinweises auf die Patenterteilung: **26.03.2014**
(21) Anmeldenummer: 04102630.3
(22) Anmeldetag: 09.06.2004
(51) Int. Cl.: A61K 8/37, A61Q 5/06, A61K 8/81, A61K 8/06

(54) **Fixierende Mehrfachgestaltung der Frisur erlaubende kosmetische Zubereitung**
Fixing cosmetic composition allowing multiple reshaping of the hair
Composition cosmétique fixante permettant plusieurs mises en forme des cheveux

(30) Priorität: 07.07.2003 DE 10330609
(43) Veröffentlichungstag der Anmeldung: 12.01.2005
(73) Patentinhaber: Beiersdorf AG, 20245 Hamburg (DE)
(72) Erfinder: Detert, Marion, 22455, Hamburg (DE); Sass, Viola, 25488, Holm (DE)

(56) Entgegenhaltungen:
- EP-A- 1 203 573
- EP-A1- 0 496 359
- WO-A-96/32093
- WO-A-98/19653
- DE-U1- 20 017 473
- GB-A- 2 242 686
- US-A- 1 852 231
- US-A- 4 342 744
- US-B1- 6 231 844
- US-B1- 6 417 238

## Beschreibung

Die vorliegende Erfindung betrifft kosmetische Zubereitungen, die ein mehrfaches Gestalten der Frisur erlauben.

Dem Anwender bekannt sind Zubereitungen, die zum einmaligen Gestalten der Frisur geeignet sind, beispielsweise Haarsprays, -gele, -öle und so fort. All diese Zubereitungen erlauben entweder das einmalige Gestalten einer Frisur wie bei Haarsprays oder Schaumfestigern, bei denen die einzelnen Haarsträhnen zum Teil mit einem Polymerfilm überzogen und miteinander verbunden werden, oder sie geben der Frisur nur wenig Halt, weil dieser die Haarsträhnen verbindende Polymerfilm fehlt, wie beispielsweise bei Gelen und Ölen. Letztgenannte Zubereitungen dienen auch einem anderen Zweck, sie sollen vor allem das Aussehen des Gesamthaares modifizieren, in dem es nass, geölt oder strähnig erscheint.

Auch Zubereitungen zur Frisurgestaltung auf Basis von Emulsionen sind an sich bekannt. Zubereitungen dieser Art können auch fixierende Polymere enthalten. Diese Produkte sollen neben der Pflege der Haare auch einen Stylingeffekt haben, sie sollen zum Modellieren der Frisur oder zum Akzentuieren einzelner Strähnen dienen. Nachteil all dieser Produkte ist jedoch, dass, bedingt durch die Formelgrundlage, die Haare beschwert werden was zu einem Verlust des Volumens der Frisur führt und den gewünschten Stylingeffekt dieser mindert. Das Haar hängt am Kopf herunter und hat ein öliges, fettiges Aussehen.

Die Schrift WO 02/09656 A2 (L'Oreal) offenbart erneut formbare Haargestaltungszubereitungen mit bestimmten, aus zwei verschiedenen Monomereinheiten aufgebauten Acrylpolymeren. Emulsionen werden als Zubereitungsform erwähnt, ein geeignetes Emulgatorsystem, dass zu Zubereitungen führt, die die gewünschten Eigenschaften bei der Anwendung aufweist, wird jedoch nicht offenbart.

Die Schrift DE 20017473 U1 (Goldwell) offenbart O/W-Haarbehandlungmittel, die ein bestimmtes Polymer und ein Gemisch aus Acrylatpolymersalz, bestimmten Alkyfettsäureester und bestimmten Fettalkoholethoxylaten enthält. Erneut formbare Haargestaltungszubereitungen werden jedoch nicht erwähnt und die beschriebenen Emulsionen weisen auch nicht solche Eigenschaften auf.

Die Schrift WO 00/21493 (Hindustan Lever) offenbart Haargestaltungszubereitungen, die zwei bestimmte Polymere, eine nichtionische oberflächenaktive Substanz mit einem HLB-Wert von mindestens 14,5 und Wasser enthalten. Dabei handelt es sich aber nicht um erneut formbare Haargestaltungszubereitungen.

Die Schrift WO 02/11684 (P&G) offenbart W/O-Haarpflegezubereitung, deren diskontinuierliche Phase bestimmte wasserlösliche Polyalkylenglycole mit einer mittleren Molmasse von 200 bis 800 g/mol enthält und deren kontinuierliche Phase einem Silikonemulgator und ein hyrophobes, flüchtiges Lösungsmittel enthält. Auch dabei handelt es sich nicht um erneut formbare Haargestaltungszubereitungen.

WO 98/19653 A (P&G) beschreibt Zubereitungen zur Gestaltung der Frisur enthaltend ein amphoteres, ein anionisches und ein kationisches Polymer.

Keine dieser Schriften offenbart, wie man Gegenstände erhalten kann, wie sie in den Ansprüchen wiedergegeben sind. Auch kann ihnen keinerlei Anregung entnommen werden, mit der man zum Gegenstand unserer Erfindung gelangen könnte.

Es hat sich für den Fachmann nicht vorhersehbar herausgestellt, dass eine kosmetische Zubereitung in Form einer O/W-Emulsion, die ein mehrfaches Gestalten einer Frisur erlaubt enthaltend mindestens ein festigendes Polymer aus der Gruppe der nichtionischen, anionischen, amphoteren oder kationischen Polymere, eine nichtionische oberflächenaktive Substanzen (B), deren HLB-Wert zwischen 3 und 6 liegt, und eine oberflächenaktive Substanz (A) aus der Gruppe der Phosphorsäureester, deren HLB-Wert zwischen 12 und 15 liegt, den Mängeln des Standes der Technik abhelfen.

Diese Zubereitungen zeichnen sich durch außerordentliche Milde und hervorragende pflegende Eigenschaften aus. Neben dem erwünschten Effekt der Pflege und der Akzentuierung einzelner Strähnen können erfindungsgemäße Zubereitungen das Haar auch mit Volumen versorgen. Die Frisur bekommt Volumen und Stand auch am Haaransatz, die Haare werden dabei nicht beschwert. Der Griff der Haare, ein weiteres Indiz für die Pflege, fühlt sich gepflegt an, ist aber nicht unangenehm fettig oder ölig und erhält dennoch die Eigenschaften eines gestalteten oder gestylten Frisur. Diese Gestaltung, auch als Styling bezeichnet, fühlt sich gut und gepflegt an - gepflegtes Aussehen und guter Griff des Haares können somit gleichzeitig erreicht werden. Überraschenderweise lässt sich im Gegensatz zu der Anwendung mit bekannten Emulsionsfomulierungen das Volumen bei Behandlung mit der beschriebenen Formelgrundlage sogar jederzeit durch Kneten der Haare mit den Fingern wieder verbessern.

Es wurde weiter gefunden, daß es dabei bevorzugt ist, wenn das festigende Polymer aus der Gruppe der nichtionischen Polymere gewählt wird. Dadurch erhält das Haar Volumen, das jederzeit auffrischbar ist, indem der Frisurträger es beispielsweise mit der Hand neu modelliert und/oder auflockert. Die Festigung des Haares fühlt sich nicht hart an, sondern flexibel und gepflegt. Die oberflächenaktive Substanz (A) wird ausgewählt aus der Gruppe der Phosphorsäureester. Weiterhin bevorzugt ist es, wenn die nichtionische oberflächenaktive Substanze (B) ausgewählt wird aus der Gruppe der Partialfettsäureester und Fettsäureester mehrwertiger Alkohole und deren ethoxylierter Derivate, besonders bevorzugt Glycerylmonostearate, Sorbitanstearate, Glycerylstearylcitrate, Sucrosestearate. Besonders bevorzugt ist es, wenn das festigende Polymer Vinylpyrrolidon als Monomereinheit enthält, bevorzug ein Homopolymer des Vinylpyrrolidon ist. Weiterhin bevorzugt ist es, wenn als oberflächenaktive Substanz (A) ein Triester des Polyethylenlaurylether und der Phosphorsäure gewählt wird. Weiter bevorzugt ist es, wenn als nichtionische oberflächenaktive Substanz (B) ein Stearinsäureester gewählt wird. Besonders bevorzugt ist es, wenn die oberflächenaktive Substanz (A) in Konzentrationen von 0,01 bis 15, bevorzugt von 0,1 bis 10, besonders bevorzugt von 0,5 bis 5 vorliegt. Weiterhin bevorzugt ist es, wenn die nichtionische oberflächenaktive Substanz (B) in Konzentrationen von 0,01 bis 15, bevorzugt von 0,1 bis 10, besonders bevorzugt von 0,5 bis 5 vorliegt. Die Erfindung umfasst auch die Verwendung einer solchen Zubereitung zum mehrfachen Gestalten einer Frisur.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Nichtionische oberflächenaktive Substanzen im Sinne der Erfindung sind nachfolgend mit ihrem HLB-Wert angegeben.

Nichtionische oberflächenaktive Substanzen (B), deren HLB-Werte im zwischen 3 und 6 liegen, können sein:

| | |
|---|---|
| 3,0: | Decaglycerindecaoleat, Decaglycerindecastearat, Decaglycerinoctaoleat, Generol 122, Glycerinmonolaurat, Mono- und Diglyceride, ethoxyliertes Propylenglycol, |
| 3-3,5: | Protegin, Protegin X, Protegin II |
| 3-4: | Glycerinmonooleat |
| 3: | Polyglycerinester |
| 3,1: | Decaglycerindecaoleat, Glycerylmonoricinoleat , Pentaervthritmonostearat, Pentaerythritsesquioleat |
| 3,2: | Ethylenglykolmonodistearat, nicht selbstemulgierend, Glykolstearat |

### Glycerinmonostearat

| | |
|---|---|
| 3,3: | Glycerindioleat, nicht selbstemulgierend |
| 3,4: | Alkylarylpolyethylenglykolether ; Glycerylmonooleat; Propylenglykolmonostearat |
| 3,5: | Amphocerin E; Ethylenglykolmonostearat; Pentaerythritmonooleat; Polyethylenglykol(100)monooleat; Propylenglykolmonofettsäureester |
| 3.6: | Ethylenglykolfettsäureester; Glycerinmonodioleat, nicht selbstemulgierend; Monoethoxylaurylether; Octylphenol, ethoxyliert; Octylphenoxypolyethoxyethanol; Ricinusöl, ethoxyliert |
| 3,7: | Dehymuls SSO; Glycerylmonostearat(-40 % Mono-); Sorbitansesquioleat |
| 3,8: | Glycerinmonodistearat, nicht selbstemulgierend; Glycerinmonooleat; Glycerinmonostearat; Polyethylenglykol(100)monostearat; Ricinusöl, hydriert |
| 4,0: | Dehymuls E; Dehymuls F; Diglycerinsesquioleat; N,N-Dimethylcaproamid; Pentaerythritmonotallat; Propylenglykolrnonolaurat; Sorbitansesquioleat |
| 4.1: | Glycerinmonostearat; Propylenglykolfettsäureester; Sorbitanmonolaurat |
| 4,3: | Dehymuls SMO; Sorbitanfettsäureester, partieller; Sorbitanmonooleat; Sorbitanmonooleat NF |
| 4,4: | 1,2-Propylenglykolnionodistearat, selbstemulgierend |
| 4,5: | Glycerinmonostearatpalmitat (90 %), nicht selbstemulgierend; Propylenglykolrnonolaurat; |

### Sorbitanmonooleat

| | |
|---|---|
| 4,6: | Glycerylmonolaurat; Isostearylalkohol, polyoxyethyliert; Nonylphenol, polyoxyethyliert; Sorbitanmonooleat |
| 4,7: | Dehymuls SMS; Diethylenglykolmonooleat; Diethylenglykolmonostearat; Sorbitanmonostearat |
| 4.8: | Pentaervthritmonolaurat |
| 4,9: | Polyoxyethylen(2)oleylalkohol; Polyoxyethylen(2)oleylether; |
| | Polyoxyethylen(2)stearylalkohol; Polyoxyethylen(2)stearylether; Ricinusöl, ethoxyliert |
| 5-7: | Glycerinmonostearat, selbstemulgierend |
| 5: | Isostearylalkohol, ethoxyliert; Sorbitanmonostearat |
| 5,0: | Ethylenglykolmonodistearat; Generol 122 E 5; Glycerinmonostearat; Polyethylenglykol(100)monoricinoleat |
| 5,0: | Polyethylenglykol(200)distearat; Polyoxyethylensorbitbienenwachs-Derivat; Sojasterin, raffmiert |
| 5,2: | Glycerinmonolaurat |
| 5,3: | Polyoxyethylencetylether |
| 5,4: | Propylenglykolstearat |
| 5,5: | Diethylenglykolmonostearat; Glycerinmonodistearat, selbstemulgierend; Polyoxyethylenmonooleat; Schmalzsaccharoglycerid |
| 5,6: | Diethylenglykolfettsäureester; Glycerinmonooleat |
| 5,9: | Polyethylenglykol(200)dilaurat; Polyoxyethylen(5)stearylalkohol |
| 6,0: | Decaglycerintetraoleat; Natriumstearoyllactylat; Natriumstearoyl-2-lactylat; |
| | Polyethylenglykol(100)monolaurat; Polyethylenglykol(200)dioleat |

Erfindungsgemäße Zubereitungen können vorteilhaft noch weitere festigende anionische, nichtionische, amphotere und kationische Polymere enthalten.

Geeignete nichtionische Polymere sind z.B. Homopolymere des Vinylpyrrolidons, z.B. Luviskol K von BASF oder Homopolymere des N-Vinylformamids z.B. PVF von National Starch.

Weitere geeignete Polymere sind Copolymereisate aus Vinylpyrrolidon und Vinylacetat z.B. Luviskol VA Typen von BASF, Terpolymere aus Vinylpyrrolidon, Vinylacetat und Propionat wie z.B. Luviskol VAP von BASF, Polyvinylcaprolactam, Polyvinylamide und deren Salze sowie Copolymere aus Vinylpyrrolidon und Dimethylaminoethylmethacrylat, Terpolymere aus Vinylcaprolactam, Vinylpyrrolidon und Dimethylaminomethacrylat; Polysiloxane und dergleichen mehr.

Geeignete kationische Polymere mit basischen Gruppen sind z.B. Copolymere von aminsubstituierten Vinylmonomeren und nicht aminsubstituierten, nicht kationischen Monomeren. Aminsubstituierte Vinylmonomere sind z.B.Dialkylaminoalkylacrylat, Dialkylaminoalkylmethacrylat, Monoalkylaminoalkylacrylat, Monoalkylaminoalkylmethacrylat, wobei die Alkylgruppen dieser Monomere vorzugsweise niedere Alkylgruppen wie z.B. C1-C7- Alkylgruppen, besonders bevorzugt C1- C3-Alkylgruppen sind.

Geeignete ammoniumsubstituierte Vinylmonomere sind z.B. Trialkylmethacryloxyalkylammonium, Trialkylacryloxyalkylammonium, Dialkyldiallylammonium und quarternäre Vinylammoniummonomere mit cyclischen, kationische Stickstoffe enthaltenden Gruppen wie Pyridinium oder Imidazolium, z.B. Alkylvinylpyridinium oder Alkylvinylimidazolium Salze. Die Alkylgruppen dieser Monomere sind vor-zugsweise niedere Alkylgruppen wie z.B. C1-C7- Alkylgruppen, besonders bevorzugt C1- C3-Alkylgruppen. Geeignete Polymere sind unter den Bezeichnungen Poly-quaternium beschriebenen Polymere wie quaternisierte Copolymere von Vinylimidazol, Vinylpyrrolidon und/oder Vinylcaprolactam (Polyquaternium-16, -44 oder -46), quaternisiertes Vinylpyrrolidon/-Dimethylaminoethylmethacrylat Copolymer (Polyquaternium-11), Homo- und Copolymere von Dimethyldiallyl-ammoniumchlorid (Polyquaternium-6, -7 oder-22), quaternisierte Hydroxy-ethylcellulose (Polyquaternium-10) oder quaternisierte Guarderivate.

Nicht aminsubstituierte, nichtkationische Comonomere sind z.B. Acrylamid, Methacrylamid, Alkyl- und Dialkylacrylamid, Alkyl- und Dialkylmethacrylamid, Acrylacrylat, Alkylmethacrylat, Vinylcaprolacton, Vinylpyrrolidon, Vinylester, Vinylalkohol, Maleinsäreanhydrid, Propylenglycol oder Ethylenglycol, wobei die Alkylgruppen dieser Monomere vorzugsweise niedere Alkylgruppen wie z.B. C1-C7-Alkylgruppen, besonders bevorzugt C1- C3-Alkylgruppen sind.

Weitere kationische Polymere sind: quaternisiertes Vinylpyrrolidon/- Dialkylaminoalkylmethacrylat Copolymere, insbesondere quaternisiertes Vinylpyrrolidon/Dimethylaminoethylmethylacrylat Copolymer (INCI: Polyquaternium-11), z.B. Gafquat 755 von ISP oder Luviquat PQ11 von BASF; Methylvinylimidazolium-chlorid/Vinylpyrrolidon Copolymer (INCI: Polyquaternium-16), z.B. die Luviquat Typen FC 370, FC 550, FC 905 oder HM552 von BASF; Methylvinylimidazolium Methylsulfat/Vinylpyrrolidon Copolymer (INCI: Polyquaternium-44), z.B. Luviquat Care oder Luviquat MS 370 von BASF; oder das Copolymer aus Vinylcaprolactam, Vinylpyrrolidon und quaternisiertem Vinylimidazol (INCI Polyquaternium-46), z.B. Luviquat Hold von BASF.

Geeignete amphotere Polymere sind Octylacrylamide/Acrylates/Butylaminoethyl Methacrylate Copolymer (Amphomer 28-4910; National Starch) oder Methacryloyl Ethylbetaine/Methacrylates Copolymer (Diaformer; Mitsubishi). Weiterhin sind geeignet Copolymerisate aus Carboxylgruppen oder Sulfongruppen enthaltenden Monomeren, z.B. (Meth)Acrylsäure und Itaconsäure mit basischen insbesondere Aminogruppen enthaltenden Monomeren wie z.B. Mono- bzw. Dialkylamino-alkyl(meth)acrylaten und/oder Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure. Geeignete anionische Polymere sind Homo- oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbareVerbindungen, welche mindestens eine Säuregruppe besitzen., insbesondere Carboxyvinylmonomere. Geeignete Säuregruppen enthaltende Monomere sind beispielsweise Acrylsäure, Meth-acrylsäure, Crotonsäure, Maleinsäure bzw. Maleinsäureanhydrid oder deren Monoester, Aldehydcarbonsäuren oder Ketocarbonsäuren.

Geeignete anionische Polymere sind Homo- oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbareVerbindungen, welche mindestens eine Säuregruppe besitzen., insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydcarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethylacrylamid, Alkylacrylat, Alkylmethacrylat, Vinylcaprolactam, Vinylpyrrolidon, Vinylester, Vinylalkohol, Propylenglycol oder Ethylenglycol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat.

Weitere Polymere mit Säuregruppen sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidonen, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden.

Andere Polymere sind: vernetzte oder unvernetzte Vinylacetat/ Crotonsäure Copolymere (INCI VA/Crotonates Copolymer) z.B. Resyn 28-1310 von National Starch oder Luviset CA66 von BASF; Terpolymere aus Vinylacetat, Crotonat und Vinylalkanoat, insbesondere Vinylacetat/Crotonat/Vinylnoedecanoat Copoly-mere (INCI: VA/Crotonates/Vinylneodecanoate Copolymer) z.B. Resyn 28-2930 von National Starch; partialveresterte Copolymere zwischen Vinylmethylether und Maleinsäureanhydrid (INCI: Ethyl-, Isopropyl-,Butylester of PVM/MA Copolymer) z.B. Gantrez ES 225 oder Gantrez ES 425 von ISP; Copolymere aus Acrylsäure oder Methacrylsäure mit Alkylacrylaten und/oder N-Alkylacrylamiden, insbesondere Copolymere aus Methacrylsäure und Alkyacrylaten sowie Terpolymeren aus Acryl-säure, Alkylacrylaten und N-Alkylacrylamiden wie Acrylsäure/Ethylacrylat/N-tert.-Butylacrylamid Terpolymer (INCI: Acrylate/Acrylamide Copolymer) z.B. Ultrahold 8 von BASF oder tert.-Butylacrylat/Ethylacrylat/ Methacrylsäure Terpolymer (INCI: Acrylates Copolymer), z.B. Luvimer von BASF; Polystyrolsulfonate (INCI: Sodium Polystyrene Sulfonate) z.B. Flexan 130 von National Starch.

Geeignete anionische Polymere sind auch in Wasser lösliche oder dispergierbare anionische Polyurethane, z.B. Luviset PUR von BASF oder Polyester.

Falls anionische Polymere verwendet werden, können als Neutralisationsmittel Hydroxide, deren Kation ein Ammonium oder ein Alkalimetall ist wie z. B. NaOH oder KOH verwendet werden. Andere Neutralisationmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM) und Dimethyl Stearamin (DMS).

Zusätzlich können pflegende Substanzen wie konditionierende Inhaltsstoffe verwendet werden., z.B. in Mengen von 0,001 bis 10 Gew.-%, bezogen auf das Gesamtgewicht der Zubereitungen.

Zu den bevorzugten Konditionierhilfsmitteln gehören kationische Polymere, die für eine Verbesserung der Pflegeeigenschaften am Haar sorgen.

Dazu gehören kationische Cellulose-Derivate synthetisiert auf Basis von Hydroxycellulose mit einem trimethylammoniumsubstituierten Epoxid. Diese Substanzen sind unter der Bezeichnung Polyquaternium-10 bekannt und kommerziell z.B. als Polymer JR 400 von der Union Carbide Cooperation erhältlich.

Weitere Substanzen z.B.: kationische Polysaccharide besonders modifizierte Guarderivate, bekannt unter der Bezeichnung JAGUAR C13S und vertrieben durch Meyhall.; Homo- und Copolymere auf Basis von (Meth)acryloyloxyethyltrimethylammoniumsalz mit dem Handelnamen Salcare SC92 oder Salcare SC95 erhältlich bei Allied Colloids; Polymere auf Basis des Monomers Diallyldimethylammoniumchlorid wie Polyquaternium-6 als Homopolymer mit dem Handelsnamen Salcare SC30 und Polyquaternium-7 als Copolymer mit Acrylamid unter dem Handelsnamen Salcare SC10; Polyquaternium-47 als Copolymer von Acrylsäure,Methacrylat und Methacrylamidopropyltrimoniumchlorid mit dem Handelsnamen Merquat 2001 N von der Firma Calgon; Copolymere von Vinylpyrrolidone und Vinylmethylimidazolium Salz wie Polyquaternium-44 erhältlich als Luviquat Care von BASF; Terpolymere von Vinylpyrrolidon, Dimethylaminopropylmethacrylamid und Alkyldimethylaminopropylmethacrylamidoammoniumsalze unter dem Handelsnamen Styleze W-20 von der Firma ISP.

Als konditionierende Öle können Polyolefine oder Mineralöle wie Hexadecan,Paraffinöl verwendet werden. Ebenso eignen sich Fettalkohole, Wachse, Paraffine, Vaseline, Paraffinöl und Lösemittel.

Fettalkohole sind z.B. gerad- oder verzweigtkettige aliphatische einwertige Alkohole mit 6 - 22 C-Atomen im Molekül. In der Kosmetik werden vorzugsweise geradkettige Fettalkohole mit einer Kettenlänge von 12 - 18 C-Atomen verwendet. Diese Fettalkohole sind weiche, farblose Massen, praktisch ungiftig und gut hautverträglich. Fettalkohole werden bevorzugt zur Herstellung von Haarkuren und Frisiercremes verwendet, wobei dem Cetylalkohol und dem Stearylalkohol besondere Bedeutung zukommt.

Wachse sind Fettsäureester, die in tierischen und pflanzlichen Produkten vorkommen, aber auch synthetisch hergestellt werden können. Das wohl bekannteste natürlich vorkommende Wachs ist das Bienenwachs, das als Hauptbestandteile Cerin und Myricin enthält. Wachs ist jedoch ein Oberbegriff für eine Reihe natürlich oder künstlich gewonnener Stoffe, die in der Regel halbfeste, weiße, geruchlose und in Wasser unlösliche Massen darstellen.

Paraffine im kosmetischen Sinn sind weiße, geruchlose Massen aus geradkettigen hochmolekularen Kohlenwasserstoffen. Wegen ihrer den der Wachse vergleichbaren Eigenschaften werden sie auch oft als Erdölwachse bezeichnet.

Vaseline ist ein Gemisch von verzweigtkettigen Paraffinen mit geringem Anteil an zyklischen Paraffinen. Es ist eine weiche, transparente und in Wasser unlösliche Masse mit geringem Eigengeruch, die bei der Aufbereitung des Erdöls anfällt.

Paraffinöl ist ein Gemisch gesättigter flüssiger Kohlenwasserstoffe. Es ist unlöslich in Wasser, aber mischbar mit Fettalkoholen und Wachsen. Es wird als Zusatz in Haarpflegemitteln zur Konsistenzregulierung verwendet.

Auch Lösemittel können vorteilhaft verwendet werden. Von der großen Anzahl Lösemittel, die zur Verfügung stehen, hat Ethanol neben Wasser die größte Bedeutung. Es wird zur Herstellung von Haarwässern verwendet, in denen es wegen seiner desinfizierenden Eigenschaften gleichzeitig die Funktion eines Wirkstoffes erfüllt.

Hilfsstoffe können verwendet werden, um bestimmte Eigenschaften der Haarpflegemittel, z.B. Konsistenz, Temperatur- und Lichtstabilität, Aussehen und Geruch, zu verbessern sowie deren Herstellung zu erleichtern. Zugesetzt werden z.B. nach Bedarf:
- Emulgatoren, um die Grenzflächenspannung zwischen zwei an sich nicht mischbaren Phasen so weit herabzusetzen, daß deren feine Vermischung möglich wird,
- Verdickungsmittel, um die Stabilität von Emulsionen zu erhöhen und deren Viskosität einzustellen,
- UV-Absorber, um die Lichtstabilität der in Haarpflegemitteln enthaltenen Farbstoffe und anderer lichtempfindlicher Komponenten zu verbessern. Daneben dienen sie dem Schutz des Haares vor Lichteinflüssen.
- Konservierungsmittel, um mikrobieller Zersetzung vorzubeugen.
- Antioxidantien, um Geruchsveränderungen, die durch Oxidationsvorgänge hervorgerufen werden können, zu verhindern.
- Farbstoffe, um Haarpflegemitteln ein ansprechendes Aussehen zu verleihen.
- Parfümöle, um Haarpflegemitteln einen angenehmen Duft zu verleihen und Nebengerüche der Rohstoffe zu überdecken.

Die Menge der Grundstoffe beträgt beispielsweise 85 bis 99,999 Gew.-%, vorzugsweise 90 bis 99,99 Gew.-%, jeweils bezogen auf das Gesamtgewicht der Zubereitung.

Quartäre Ammonium-Verbindungen sind eine wichtige Gruppe der speziellen Wirkstoffe, die zur Herstellung von Haarpflegemitteln verwendet werden. Haarpflegemittel, insbesondere Haarkuren, erhalten wesentliche Eigenschaften wie Verbesserung von Kämmbarkeit sowie Griff und Verhinderung statischer Aufladung der Haare vornehmlich durch den Einsatz quartärer Ammonium-Verbindungen.

Die Eigenschaften quartärer Ammonium-Verbindungen werden durch die kationische Gruppe einerseits und durch die Art der lipophilen Reste dieser Gruppe andererseits bestimmt. Geeignet sind z.B. die Verbindungen, in denen ein bis zwei Reste längerkettige Alkyl-Gruppen, wie Lauryl-, Cetyl- oder Stearyl-Gruppen, und die verbliebenen Reste Methyl-Gruppen sind. Produkte dieses Typs werden vorzugsweise als Chloride, Bromide und Methosulfate eingesetzt.

Geeignet sind auch polymere quartäre Ammonium-Verbindungen, Makromoleküle, deren wesentliches Merkmal das Vorhandensein mehrerer quartärer AmmoniumGruppen im Molekül ist. Dadurch wird ihre Haftfähigkeit am Haar deutlich erhöht.

Besonders vorteilhaft zu verwendende kationische Tenside sind
1. Alkylamine
2. Alkylimidazole
3. Ethoxylierte Amine und
4. Quaternäre Tenside.
5. Esterquats

Quaternäre Tenside erhalten mindestens ein N-Atom, das mit 4 Alkyl- oder Arylgruppen kovalent verbunden ist. Dies führt, unabhängig vom pH-Wert, zu einer positiven Ladung. Die erfindungsgemäß verwendeten kationischen Tenside können ferner bevorzugt gewählt werden aus der Gruppe der quaternären Ammoniumverbindungen, insbesondere Benzyltrialkylammoniumchlorid, ferner Alkyltrialkylammoniumsalze, beispielsweise Cetyltrimethylammoniumchlorid oder - bromid, Alkyldimethylhydroxyethylammoniumchlorideoder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Monomere oder polymere quartäre Ammonium-Verbindungen werden vielfach in Haarspülungen und Haarkuren z.B. in Konzentrationen von 0,5 - 5 Gew.-% eingesetzt. Dazu gehören Cetrimonium Chloride wie es unter der Bezeichnung Dehyquart A von der Fa. Henkel angeboten wird oder Distearoylethyl Hydroxyethylmonium Methosulfate wie es unter der Bezeichnung Dehyquart F 75 von der Fa. Henkel angeboten wird.

Erfindungsgemäß können kosmetische Zubereitungen zur Behandlung und Pflege der Haare als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose; Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die vorstehenden Prozentangaben beziehen sich auf das Gesamtgewicht der Zubereitungen.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Auch Vitamine, Proteine und deren Derivate, feuchtigkeitsspendende Substanzen, Extrakte, Parfüms, Adhäsionsverminderer können in erfindungsgemäßen Zubereitungen vorteilhaft verwendet werden.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die Menge der Verdickungsmittel beträgt beispielsweise 0,05 bis 5,0 Gew.-%, vorzugsweise 0,1 bis 3,0 Gew.-%, insbesondere 0,15 bis 2,0 Gew.-%, jeweils bezogen auf das Gesamtgewicht der Zubereitung.

Der Wassergehalt der Zubereitungen beträgt beispielsweise 60 bis 95 Gew.-%, vorzugsweise 75 bis 95 Gew.-%, insbesondere 80 bis 90 Gew.-%, jeweils bezogen auf das Gesamtgewicht der Zubereitung.

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

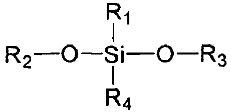

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 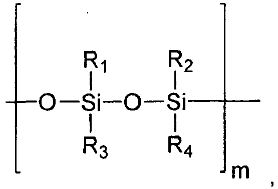 wobei die Siliciumatome mit gleichen oder unterschiedlichen Alkylresten und/oder Arylresten substituiert werden können, welche hier verallgemeinernd durch die Reste R1 - R4 dargestellt sind (will sagen, daß die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 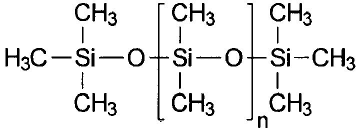 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

| Hersteller | Handelsname | INCI-Name | Polarität [mN/m] |
|---|---|---|---|
| Wacker | Wacker Silikonöl AK 100 | Polydimethylsiloxan | 26,9 |
| Wacker | Wacker Silikonöl AK 50 | Polydimethylsiloxan | 46,5 |
| Wacker | Wacker Silikonöl AK 35 | Polydimethylsiloxan | 42,4 |
| Wacker | Wacker Silikonöl AK 20 | Polydimethylsiloxan | 40,9 |
| Dow Corning | Dow Corning Fluid 245 | Cyclopentasiloxan | 32,3 |
| Dow Corning | Dow Corning Fluid 345 | Cyclomethicone | 28,5 |

Erfindungsgemäß vorteilhaft einzusetzende cyclische Silicone werden im allgemeinen durch Strukturelemente charakterisiert, wie folgt 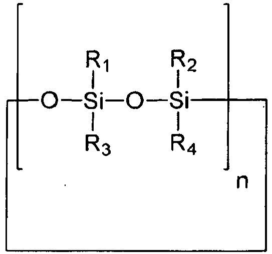 wobei die Siliciumatome mit gleichen oder unterschiedlichen Alkylresten und/oder Arylresten substituiert werden können, welche hier verallgemeinernd durch die Reste R1 - R4 dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methyl-phenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyether-copolymere wie das Cetyl-Dimethicon-Copolyol sowie das Cetyl-Dimethicon-Copolyol (und) Polyglyceryl-4-Isostearat (und) Hexyllaurat.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A-bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO2), Zinks (ZnO), Eisens (z. B. Fe2O3), Zirkoniums (ZrO2), Siliciums (SiO2), Mangans (z. B. MnO), Aluminiums (Al2O3), Cers (z. B. Ce2O3), Mischoxide der entsprechenden Metalle sowie Abmischungen aus solchen Oxiden, sowie das Sulfat des Bariums (BaSO4).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form commerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 150 nm aus.

| Handelsname | Coating | zusätzliche Bestandteile der Vordispersion | Hersteller |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure | - | Merck KgaA |
| Titandioxid P25 | None | - | Degussa |
| Titandioxid T805 (Uvinul TiO2) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 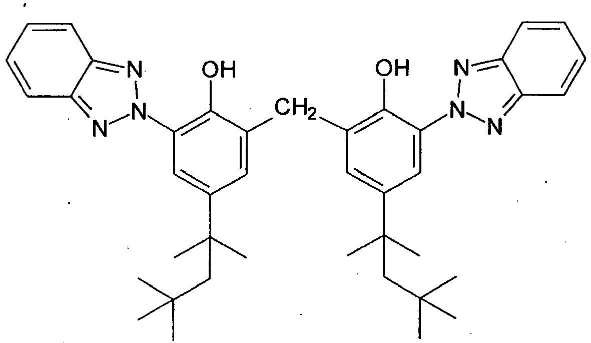 gekennzeichnet ist und unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 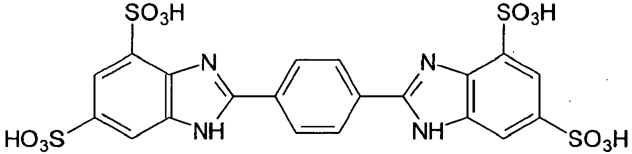 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 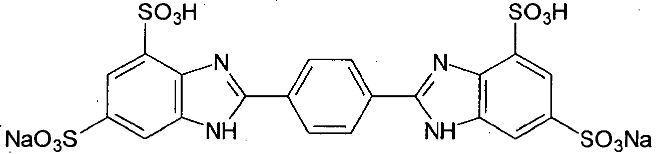 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

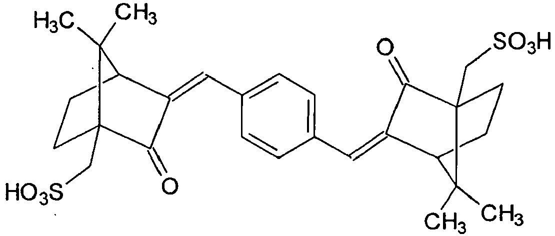

Weitere vorteilhafte UV-A-Filtersubstanzen sind Hydroxybenzophenone, die sich durch die folgende Strukturformel auszeichnen: 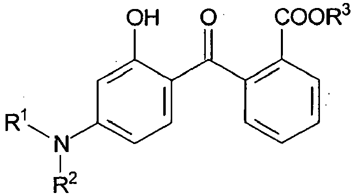 worin
- R1 und R2 unabhängig voneinander Wasserstoff, C1-C20-Alkyl, C3-C10-Cycloalkyl oder C3-C10-Cycloalkenyl bedeuten, wobei die Substituenten R1 und R2 gemeinsam mit dem Stickstoffatom, an das sie gebunden sind, einen 5- oder 6-Ring bilden können und
- R3 einen C1-C20-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 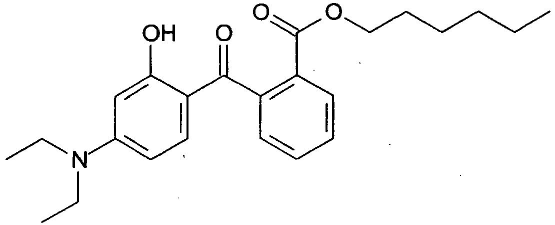 und unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 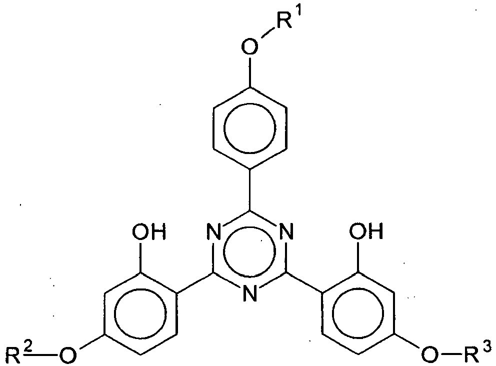 wobei R1, R2 und R3 unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 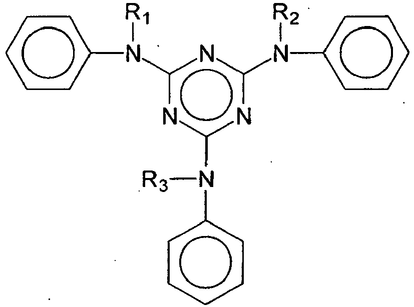 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 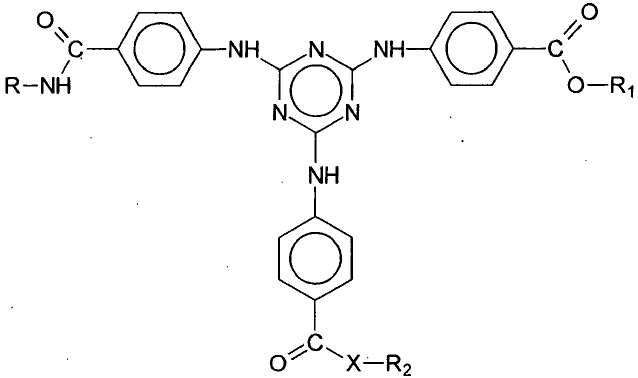 wiedergegeben wird, wobei
- R: einen verzweigten oder unverzweigten C1-C18-Alkylrest, einen C5-C12-Cycloalkylrest, gegebenenfalls substituiert mit einer oder mehreren C1-C4- Alkylgruppen, darstellt,
- X: ein Sauerstoffatom oder eine NH-Gruppe darstellt,
- R1: einen verzweigten oder unverzweigten C1-C18-Alkylrest, einen C5-C12-Cycloalkylrest, gegebenenfalls substituiert mit einer oder mehreren C1-C4- Alkylgruppen, oder ein Wasserstoffatom, ein Alkalimetallatom, eine Ammoniumgruppe oder eine Gruppe der Formel 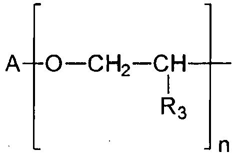 bedeutet, in welcher
- A: einen verzweigten oder unverzweigten C1-C18-Alkylrest, einen C5-C12-Cycloalkyl- oder Arylrest darstellt, gegebenenfalls substituiert mit einer oder mehreren C1-C4- Alkylgruppen,
- R3: ein Wasserstoffatom oder eine Methylgruppe darstellt,
- n: eine Zahl von 1 bis 10 darstellt,
- R2: einen verzweigten oder unverzweigten C1-C18-Alkylrest, einen C5-C12-Cycloalkylrest, gegebenenfalls substituiert mit einer oder mehreren C1-C4- Alkylgruppen, darstellt, wenn X die NH-Gruppe darstellt, und
einen verzweigten oder unverzweigten C1-C18-Alkylrest, einen C5-C12-Cycloalkylrest, gegebenenfalls substituiert mit einer oder mehreren C1-C4- Alkylgruppen, oder ein Wasserstoffatom, ein Alkalimetallatom, eine Ammoniumgruppe oder eine Gruppe der Formel 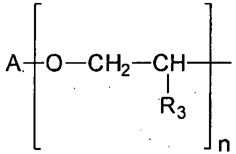 bedeutet, in welcher
- A: einen verzweigten oder unverzweigten C1-C18-Alkylrest, einen C5-C12-Cycloalkyl- oder Arylrest darstellt, gegebenenfalls substituiert mit einer oder mehreren C1-C4- Alkylgruppen,
- R3: ein Wasserstoffatom oder eine Methylgruppe darstellt,
- n: eine Zahl von 1 bis 10 darstellt,
- wenn X: ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 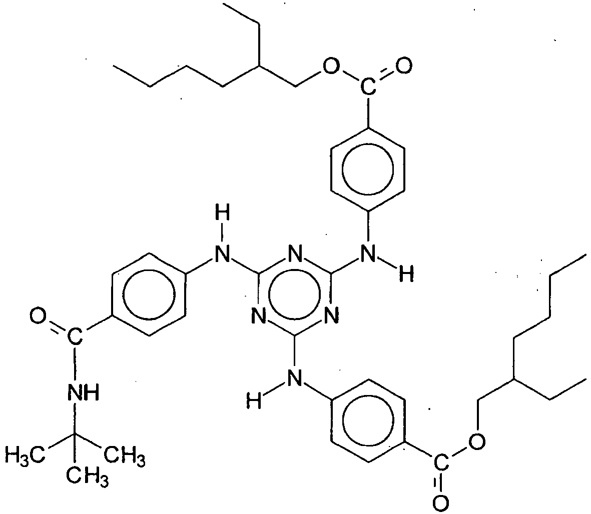 wiedergegeben wird, welches im Folgenden auch als Dioctylbutylamidotriazon (INCI: Dioctylbutamidotriazone) bezeichnet wird und unter der Handelsbezeichnung UVA-SORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 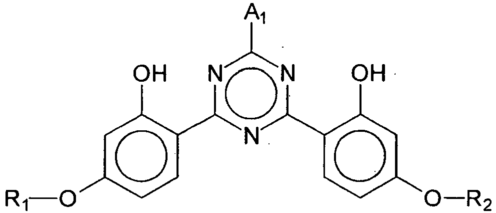 wiedergegeben wird, wobei R1 , R2 und A1 verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenyl-amino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 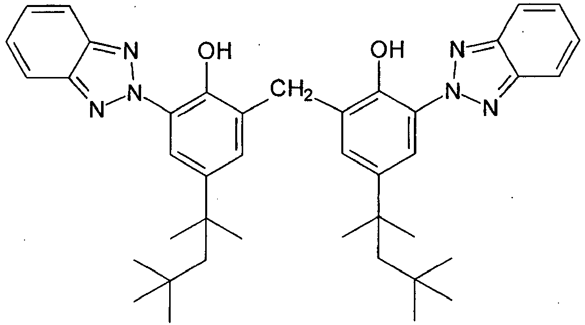 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 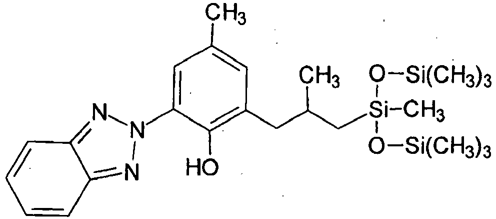 gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornyliden-methyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/ Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

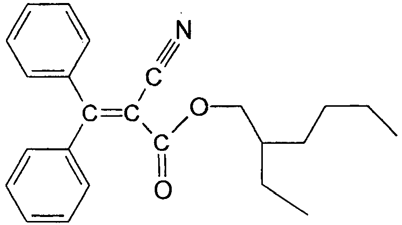

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Es kann auch gegebenenfalls vorteilhaft im Sinne der vorliegenden Erfindung sein, den Zubereitungen gemäß der Erfindung Farbstoffe und/oder -pigmente einzuverleiben.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe2O3, Fe3O4, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxy-benzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C30) | 40820 | orange |
| trans-Apo-8'-Carotinsäure (C30)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)A2,5-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylamino-naphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268: 1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn3(PO4)2 · 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1' phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 8-Amino-2 -phenylazo- 1 -naphthol-3,6-disulfosäure , Aluminiumsalz der 4-(4-Sulfo-1-phenylazo)-1-(4-sulfophenyl)-5-hydroxy-pyrazolon-3-carbonsäure, 4'-[(4"-Sulfo-1"-phenylazo)-7'-sulfo-1'-naphthylazo]-1-hydroxy-8-acetyl-aminonaphthalin-3,5-disulfosäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, 4,4'-Dimethyl-6,6' dichlorthioindigo, Komplexsalz (Na, Al, Ca) der Karminsäure, rotes und schwarzes Eisenoxid (CIN: 77 491 (rot) und 77 499 (schwarz)), Eisenoxidhydrat (CIN: 77 492), Manganammoniumdiphosphat (CIN 77745), Ultramarin (CIN 77007) und Titandioxid.

Ferner vorteilhaft sind öllösliche Naturfarbstoffe, wie z. B. Paprikaextrakte, □-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:
Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
Monokristalline Perlglanzpigmente wie z. B. Bismuthoxychlorid (BiOCl)
Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | TiO2: 40 - 60 nm | silber |
| Interferenzpigmente | TiO2: 60 - 80 nm | gelb |
| | TiO2: 80 - 100 nm | rot |
| | TiO2: 100 - 140 nm | blau |
| | TiO2: 120 - 160 nm | grün |
| Farbglanzpigmente | Fe2O3 | bronze |
| | Fe2O3 | kupfer |
| | Fe2O3 | rot |
| | Fe2O3 | rotviolett |
| | Fe2O3 | rotgrün |
| | Fe2O3 | schwarz |
| Kombinationspigmente | TiO2 / Fe2O3 | Goldtöne |
| | TiO2 / Cr2O3 | grün |
| | TiO2 / Berliner Blau | tiefblau |
| | TiO2 / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO2 und Fe2O3 beschichtete SiO2-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von SiO2 hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 □m zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 20 Gew.-%, insbesondere von 1,0 bis 15 Gew.-% gewählt, jeweils bezogen auf das Gesamtgewicht der Zubereitungen.

Vorteilhaft werden die Antioxidantien gewählt aus der Gruppe bestehend aus Aminosäuren (z.B. Glycin, Lysin, Arginin, Cystein, Histidin, Tyrosin, Tryptophan) und deren Derivate (als Salz-, Ester-, Ether-, Zucker-, Nukleotid-, Nukleosid-, Peptid- und Lipid-Verbidung), Imidazole (z.B. Urocaninsäure) und deren Derivate (als Salz-, Ester-, Ether-, Zucker-, Nukleotid-, Nukleosid-, Peptid- und/oder Lipid-Verbidung), Peptide wie D,L-Carnosin, D-Carnosin, L-Carnosin, Anserin und deren Derivate (z.B. als Salz-, Ester-, Ether-, Zucker-, Thiol-, Nukleotid-, Nukleosid-, Peptid- und Lipid-Verbidung), Carotinoide, Carotine (z.B. α-Carotin, β-Carotin, ψ-Lycopin, Phytoen, ) und deren Derivate (z. B. als Salz-, Ester-, Ether-, Zucker-, Nukleotid-, Nukleosid-, Peptid- und/oder Lipid-Verbidung), Chlorogensäure und deren Derivate (als Salz-, Ester-, Ether-, Zucker-, Thiol-, Nukleotid-, Nukleosid-, Peptid- und/oder Lipid-Verbidung), Aurothioglucose, Propylthiouracil und andere Thiole (z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (als Salz-, Ester-, Ether-, Zucker-, Thiol-, Nukleotid-, Nukleosid-, Peptid- und/oder Lipid-Verbidung) sowie Sulfoximinverbindungen (z.B. Homocysteinsulfoximin, Buthioninsulfone, Penta-, Hexa-, Heptathioninsulfoximin) in sehr geringen verträglichen Dosierungen (z.B. pmol bis µmol/kg). Ferner (Metall)-Chelatoren (z.B. Apoferritin, Desferral, Lactoferrin, α-Hydroxyfettsäuren, Palmitinsäure, Phytinsäure) und deren Derivate (als Salz-, Ester-, Ether-, Zucker-, Thiol-, Nukleotid-, Nukleosid-, Peptid- und/oder Lipid-Verbidung), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon, Ubichinol, Plastochinon und deren Derivate (als Salz-, Ester-, Ether-, Zucker-, Thiol-, Nukleotid-, Nukleosid-, Peptid- und Lipid-Verbidung), Vitamin C und Derivate (z.B. Ascorbylpalmitat, Mg-Ascorbylphosphat, Ascorbylacetat), Tocopherole und Derivate (z.B. Vitamin-E-acetat), sowie Phenolische Verbindungen und Pflanzenextrakte, diese enthaltend, wie z. B. Flavonoide (z. B. Glycosylrutin, Ferulasäure, Kaffeesäure), Furfurylidenglucitol, Butylhydroxytoluol, Butylhydroxyanisol, Nordihydroguajakharzsäure, Nordihydroguajaretsäure, Trihydroxybutyrophenon und deren Derivate (als Salz-, Ester-, Ether-, Zucker-, Nukleotid-, Nukleosid-, Peptid- und Lipid-Verbidung). Harnsäure und deren Derivate, Mannose und deren Derivate (als Salz-, Ester-, Ether-, Zucker-, Thiol-, Nukleotid-, Nukleosid-, Peptid- und Lipid-Verbidung). Zink und dessen Derivate (z.B. ZnO, ZnSO4), Selen und dessen Derivate (z.B. Selenmethionin, Ebselen), Stilbene und deren Derivate (z.B. Stilbenoxid, Trans-Stilbenoxid) und die erfindungsgemäß geeigneten Derivate (als Salz-, Ester-, Ether-, Zucker-, Thiol-, Nukleotid-, Nukleosid-, Peptid- und/oder Lipid-Verbidung) dieser genannten Wirkstoffe.

In der Lebensmitteltechnologie zugelassene Konservierungsmittel, die mit ihrer E-Nummer nachfolgend aufgeführt sind, sind erfindungsgemäß vorteilhaft zu verwenden.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylpheno lat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konervierungsmittel oder Konservierungshilfsstoffe Dibromdicyanobutan (2-Brom-2-brommethylglutarodinitril), 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, DMDM Hydantoin , IPBC (Formaldehydabspalter) geeignet.

Ferner sind Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel geeignet.

Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1 ,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Geeignete Verdicker sind:
Homopolymere der Acrylsäure mit einem Molekulargewicht von 2000000 bis 6000000 wie z.B. Handelsprodukt Carbopole. Weiter Verdicker werden vertrieben unter den Namen Carbopol 940, Carbopol EDTA 2001 oder Modarez V 600 PX .
Polymere aus Acrylsäure und Acrylamid (Natriumsalz) mit einem Molekulargewicht von 2000000 bis 6000000 wie z.B. Hostacerin PN 73 oder das unter dem Namen Amigel vertriebene Sclerotium Gum.

Außerdem geeignet sind Copolymere der Acrylsäure oder der Methacrylsäure wie z.B. Carbopol 1342 oder Permulen TRI.

Weitere Verdickertypen sind Polyglycole, Cellulosederivate, insbesondere Hydroxyalkylcellulosen sowie Alginate, Carageenan und anorganische Verdicker wie z.B. natürliche oder synthetische Bentonite.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

| | Styling Creme | | Styling Creme | |
|---|---|---|---|---|
| | **9** | **10** | **11** | **12** |
| Paraffinöl | 5,0 | 5,0 | 5,0 | 5,0 |
| PEG-2 Oleat | 3,0 | 2,0 | 3,0 | 2,0 |
| Trilaureth-4 Phosphat | 1,5 | 2,2 | 1,5 | 2,2 |
| PVP/DMPAPA Acrylat Copolymer | 1,5 | 1,3 | | |
| Sodium Polystyrene Sulfonat | | | 4,0 | 3,5 |
| Glycerin | 5,0 | 6,0 | 5,0 | 6,0 |
| Polyethylenglycol | 1,0 | 2,0 | 2,0 | 2,0 |
| Konservierungsmittel | 0,4 | 0,4 | 0,4 | 0,4 |
| Parfüm | 0,2 | 0,2 | 0,2 | 0,2 |
| Wasser | Ad 100 | Ad 100 | Ad 100 | Ad 100 |

| | Styling Creme | | Styling Creme | |
|---|---|---|---|---|
| | **13** | **14** | **15** | **16** |
| Cetyl Alkohol | 5,0 | 5,0 | 5,0 | 5,0 |
| Sorbitan Stearat | 3,0 | 2,0 | 3,0 | 2,0 |
| Trilaureth-4 Phosphat | 1,5 | 2,2 | 1,5 | 2,2 |
| PVP | 1,5 | 1,3 | | |
| Polyquaternium-46 | | | 4,0 | 3,5 |
| Glycerin | 5,0 | 6,0 | 5,0 | 6,0 |
| Konservierungsmittel | 0,4 | 0,4 | 0,4 | 0,4 |
| Parfüm | 0,2 | 0,2 | 0,2 | 0,2 |
| Wasser | Ad 100 | Ad 100 | Ad 100 | Ad 100 |

## Patentansprüche

1. Kosmetische Zubereitung in Form einer O/W-Emulsion, die ein mehrfaches Gestalten einer Frisur erlaubt enthaltend
- mindestens ein festigendes Polymer aus der Gruppe der nichtionischen, anionischen, amphoteren oder kationischen Polymere,
- eine nichtionische oberflächenaktive Substanz (B), deren HLB-Wert zwischen 3 und 6 liegt, und
- eine oberflächenaktive Substanz (A) ausgewählt aus der Gruppe der Phosphorsäureester, deren HLB-Wert zwischen 12 und 15 liegt.

2. Zubereitung nach Anspruch 1 **dadurch gekennzeichnet, dass** das festigende Polymer aus der Gruppe der nichtionischen Polymere gewählt wird

3. Zubereitung nach einem der vorangehenden Ansprüche, **dadurch gekennzeichnet, dass** die nichtionische oberflächenaktive Substanz (B) ausgewählt wird aus der Gruppe der Partialfettsäureester und Fettsäureester mehrwertiger Alkohole und deren ethoxylierter Derivate, besonders bevorzugt Glycerylmonostearate, Sorbitanstearate, Glycerylstearylcitrate, Sucrosestearate.

4. Zubereitung nach einem der vorangehenden Ansprüche, **dadurch gekennzeichnet, dass** das festigende Polymer Vinylpyrrolidon als Monomereinheit enthält, bevorzug ein Homopolymer des Vinylpyrrolidon ist.

5. Zubereitung nach einem der vorangehenden Ansprüche, **dadurch gekennzeichnet, dass** als oberflächenaktive Substanz (A) ein Triester des Polyethylenlaurylether und der Phosphorsäure gewählt wird.

6. Zubereitung nach einem der vorangehenden Ansprüche, **dadurch gekennzeichnet, dass** als nichtionische oberflächenaktive Substanz (B) ein Stearinsäureester gewählt wird.

7. Zubereitung nach einem der vorangehenden Ansprüche, **dadurch gekennzeichnet, dass** die oberflächenaktive Substanz (A) in Konzentrationen von 0,01 bis 15, bevorzugt von 0,1 bis 10, besonders bevorzugt von 0,5 bis 5 vorliegt.

8. Zubereitung nach einem der vorangehenden Ansprüche, **dadurch gekennzeichnet, dass** die nichtionische oberflächenaktive Substanz (B) in Konzentrationen von 0,01 bis 15, bevorzugt von 0,1 bis 10, besonders bevorzugt von 0,5 bis 5 vorliegt.

9. Verwendung einer Zubereitung nach einem der vorangehenden Ansprüche zum ein- oder mehrfachen Gestalten einer Frisur.

## Claims

1. Cosmetic preparation in the form of a O/W emulsion which allows multiple shaping of a hairstyle, comprising
- at least one setting polymer from the group of nonionic, anionic, amphoteric or cationic polymers,
- a nonionic surface-active substance (B) whose HLB value is between 3 and 6, and
- a surface-active substance (A) selected from the group of phosphoric acid esters whose HLB value is between 12 and 15.

2. Preparation according to Claim 1, **characterized in that** the setting polymer is selected from the group of nonionic polymers.

3. Preparation according to one of the preceding claims, **characterized in that** the nonionic surface-active substance (B) is selected from the group of partial fatty acid esters and fatty acid esters of polyhydric alcohols and their ethoxylated derivatives, particularly preferably glyceryl monostearates, sorbitan stearates, glyceryl stearyl citrates, sucrose stearates.

4. Preparation according to one of the preceding claims, **characterized in that** the setting polymer comprises vinylpyrrolidone as monomer unit, is preferably a homopolymer of vinylpyrrolidone.

5. Preparation according to one of the preceding claims, **characterized in that** a triester of polyethylene lauryl ether and of phosphoric acid is selected as surface-active substance (A).

6. Preparation according to one of the preceding claims, **characterized in that** a stearic acid ester is selected as nonionic surface-active substance (B).

7. Preparation according to one of the preceding claims, **characterized in that** the surface-active substance (A) is present in concentrations of from 0.01 to 15, preferably from 0.1 to 10, particularly preferably from 0.5 to 5.

8. Preparation according to one of the preceding claims, **characterized in that** the nonionic surface-active substance (B) is present in concentrations of from 0.01 to 15, preferably from 0.1 to 10, particularly preferably from 0.5 to 5.

9. Use of a preparation according to one of the preceding claims for the single or multiple shaping of a hairstyle.

## Revendications

1. Préparation cosmétique sous forme d'une émulsion H/E, qui permet un coiffage multiple d'une coiffure, contenant
- au moins un polymère fixateur du groupe des polymères non ioniques, anioniques, amphotères ou cationiques,
- une substance tensioactive non ionique (B), dont la valeur BHL est située entre 3 et 6, et
- une substance tensioactive (A) choisie dans le groupe des esters de l'acide phosphorique, dont la valeur BHL est située entre 12 et 15.

2. Préparation selon la revendication 1, **caractérisée en ce que** le polymère fixateur est choisi dans le groupe des polymères non ioniques.

3. Préparation selon l'une quelconque des revendications précédentes, **caractérisée en ce que** la substance tensioactive non ionique (B) est choisie dans le groupe des esters partiels d'acides gras et des esters des acides gras d'alcools polyvalents et leurs dérivés éthoxylés, de manière particulièrement préférée le monostéarate de glycéryle, le stéarate de sorbitane, le citrate de glycéryl-stéaryle, le stéarate de saccharose.

4. Préparation selon l'une quelconque des revendications précédentes, **caractérisée en ce que** le polymère fixateur contient de la vinylpyrrolidone comme unité monomère, est de préférence un homopolymère de la vinylpyrrolidone.

5. Préparation selon l'une quelconque des revendications précédentes, **caractérisée en ce qu'**on choisit, comme substance tensioactive (A), un triester du polyéthylènelauryléther et de l'acide phosphorique.

6. Préparation selon l'une quelconque des revendications précédentes, **caractérisée en ce qu'**on utilise comme substance tensioactive non ionique (B) un ester de l'acide stéarique.

7. Préparation selon l'une quelconque des revendications précédentes, **caractérisée en ce que** la substance tensioactive (A) se trouve en des concentrations de 0,01 à 15, de préférence de 0,1 à 10 et de manière particulièrement préférée de 0,5 à 5.

8. Préparation selon l'une quelconque des revendications précédentes, **caractérisée en ce que** la substance tensioactive non ionique (B) se trouve en des concentrations de 0,01 à 15, de préférence de 0,1 à 10 et de manière particulièrement préférée de 0,5 à 5.

9. Utilisation d'une préparation selon l'une quelconque des revendications précédentes pour le coiffage unique ou multiple d'une coiffure.
